(19) Europäisches Patentamt
European Patent Office
Office européen des brevets

(11) Veröffentlichungsnummer : **0 051 762**
**B1**

# (12) EUROPÄISCHE PATENTSCHRIFT

(45) Veröffentlichungstag der Patentschrift : **19.12.84**

(21) Anmeldenummer : **81108466.4**

(22) Anmeldetag : **17.10.81**

(51) Int. Cl.$^3$ : **C 07 J 1/00**, A 61 K 31/565// C07J21/00, C12P33/06

## (54) 11-Methylen-delta-15-Steroide, Verfahren zu ihrer Herstellung und diese enthaltende pharmazeutische Präparate.

(30) Priorität : **07.11.80 DE 3042529**

(43) Veröffentlichungstag der Anmeldung : **19.05.82 Patentblatt 82/20**

(45) Bekanntmachung des Hinweises auf die Patenterteilung : **19.12.84 Patentblatt 84/51**

(84) Benannte Vertragsstaaten : **AT BE CH DE FR GB IT LI LU NL SE**

(56) Entgegenhaltungen :
**EP-A- 0 017 094**
**DE-A- 2 361 120**
**FR-A- 2 326 927**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT Berlin und Bergkamen Müllerstrasse 170/178 Postfach 65 03 11 D-1000 Berlin 65 (DE)**

(72) Erfinder : **Hofmeister, Helmut, Dr. Weislingenstrasse 4 D-1000 Berlin 28 (DE)**
Erfinder : **Petzoldt, Karl, Dr. Flachsweg 10 D-1000 Berlin 38 (DE)**
Erfinder : **Annen, Klaus, Dr. Seegefelderstrasse 194 D-1000 Berlin 20 (DE)**
Erfinder : **Laurent, Henry, Dr. Glambecker Weg 21 D-1000 Berlin 28 (DE)**
Erfinder : **Wiechert, Rudolf, Prof. Dr. Petzower Strasse 8 A D-1000 Berlin 39 (DE)**
Erfinder : **Steinbeck, Hermann, Dr. Hammerstrasse 46 D-1000 Berlin 37 (DE)**

**EP 0 051 762 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 11-Methylen-$\Delta^{15}$-Steroide, Verfahren zu ihrer Herstellung und diese enthaltende pharmazeutische Präparate.

Die Acylgruppen $R^1$ gemäß Formel I leiten sich von Säuren ab, die in der Steroidchemie üblicherweise für Veresterungen angewendet werden. Bevorzugte Säuren sind organische Carbonsäuren mit bis zu 15 Kohlenstoffatomen, insbesondere niedere und mittlere aliphatische Carbonsäuren mit bis zu 7 Kohlenstoffatomen. Die Säuren können auch ungesättigt, verzweigt, mehrbasisch oder in üblicher Weise, zum Beispiel durch Hydroxy-, Acyloxy-, Alkoxy-, Oxo- oder Aminogruppen oder Halogenatome, substituiert sein. Geeignet sind auch cycloaliphatische, aromatische, gemischt aromatisch-aliphatische und heterocyclische Säuren, die ebenfalls in üblicher Weise substituiert sein können.

Beispielsweise seien folgende Carbonsäuren genannt : Ameisensäure, Essigsäure, Propionsäure, Buttersäure, Isobuttersäure, Valeriansäure, Isovaleriansäure, Capronsäure, Önanthsäure, Cyprylsäure, Pelargonsäure, Caprinsäure, Undecylsäure, Laurinsäure, Tridecylsäure. Myristinsäure, Pentadecylsäure, Trimethylessigsäure, Diethylessigsäure, tert.-Butylessigsäure, β-Cyclopentylpropionsäure, Cyclohexylessigsäure, Cyclohexancarbonsäure, Phenylessigsäure, Phenoxyessigsäure, Mono-, Di- und Trichloressigsäure, Aminoessigsäure, Diethylaminoessigsäure, Piperidinoessigsäure, Morpholinoessigsäure, Milchsäure, O-Tridecanoylglykolsäure, Bernsteinsäure, Adipinsäure, Benzoesäure, Nikotinsäure, Isonikotinsäure, Furan-2-carbonsäure.

Die neuen 11-Methylen-$\Delta^{15}$-Steroide der allgemeinen Formel I werden gemäß Anspruch 11 durch Einführung des Restes $R^2$ in 18-Methyl-11-methylen-4,15-östradien-17-on der Formel II hergestellt.

Die Einführung des Restes $R^2$ kann nach bekannten Methoden mit einer metallorganischen Ethinyl-, Chlorethinyl- oder Propinylverbindung vorgenommen werden. Solche metallorganischen Verbindungen sind zum Beispiel Alkalimetallacetylide, wie zum Beispiel Kalium- und Lithiumacetylid, -chloracetylid bzw. -methylacetylid.

Die metallorganische Verbindung kann auch *in situ* gebildet und mit dem 17-Keton der Formel II zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines $C_4$- oder $C_5$-Alkohols oder von Ammoniak oder in Form von zum Beispiel Butyllithium einwirken lassen. Lithiumchloracetylid kann aus 1,2-Dichlorethylen und einer etherischen Lithiummethyllösung gebildet werden.

Als metallorganische Ethinylverbindungen sind auch Ethinylmagnesium- oder Ethinylzinkhalogenide, insbesondere Ethinylmagnesiumbromid oder -jodid, geeignet.

Als Lösungsmittel sind Dialkylether, Tetrahydrofuran, Dioxan, Benzol, Toluol usw., geeignet.

Die sich gegebenenfalls anschließende Veresterung der 17-Hydroxygruppe erfolgt nach Methoden, die man üblicherweise in der Steroidchemie zur Veresterung tertiärer Hydroxygruppen anwendet. Als geeignete Veresterungsmethode sei beispielsweise die Umsetzung der Steroide mit Säureanhydriden oder Säurechloriden in Gegenwart basischer Katalysatoren, wie Natriumhydrogenkarbonat, Kaliumhydrogenkarbonat, Kaliumkarbonat, Natriumhydroxid, Kaliumhydroxid, Pyridin, Lutidin, Collidin, Triethylamin oder 4-Dimethylaminopyridin, genannt. Nach einer bevorzugten Ausführungsform wird die Veresterung in Gegenwart von Pyridin und 4-Dimethylaminopyridin durchgeführt.

Die Hydrolyse der 3-Oxoschutzgruppe, die vor oder auch nach der möglichen Veresterung erfolgen kann, wird nach den dem Fachmann bekannten Methoden durchgeführt. Für die Hydrolyse kommen beispielsweise Mineralsäuren, wie zum Beispiel Perchlorsäure, Schwefelsäure oder Salzsäure, oder organische Säuren, wie zum Beispiel Oxalsäure, in Betracht. Die Hydrolyse wird vorzugsweise in alkoholischer Lösung oder in anderen polaren Lösungsmitteln, wie zum Beispiel Aceton, bei Temperaturen zwischen etwa 20 und 100 °C durchgeführt.

Die Oxoschutzgruppe X in Formel II bildet mit der (den) Doppelbindung(en), welche im Ring A oder B vorhanden sind, eine solche Anordnung von Atomen, daß die Verbindung durch saure Hydrolyse in ein 4,5-ungesättigtes 3-Oxosteroid umgewandelt wird. Nach einer bevorzugten Ausführungsform is die 3-Oxogruppe durch Ketalbildung geschützt.

Die Ketalreste leiten sich von den üblicherweise zum Schutz freier Oxogruppen verwendeten Alkoholen und Thioalkoholen ab, beispielsweise genannt seien : Ethylenglykol, 2,2-Dimethyl-propandiol-(1,3) und Ethandithiol-(1,2). Die 3-Oxogruppe kann aber auch durch Enoläther-, Enolester- oder Enaminbildung partiell geschützt werden.

Es ist bereits bekannt, daß 11-Methylensteroide wertvolle biologische Eigenschaften besitzen. In der deutschen Offenlegungsschrift 2 361 120 werden zum Beispiel 11-Methylen-17α-ethinyl-18-methyl-4-östrene beschrieben, die eine starke gestagene Wirkung zeigen.

11-Methylensteroide gemäß DE-A-2 361 120 besitzen geringere androgene Nebenwirkung und $\Delta^{15}$-Steroide gemäß FR-A-2 326 927 stärkere gestagene Hauptwirkung als die entsprechenden Steroide ohne Methylengruppe bzw. ohne $\Delta^{15}$-Doppelbindung. Eine Überprüfung ergab, daß sowohl die 11-Methylen- als auch die $\Delta^{15}$-Verbindung noch eine geringe androgene Nebenwirkung zeigen.

Eine deutliche Abschwächung der androgenen Nebenwirkung wurde bereits früher bei 17α-Chlorethinylsteroiden im Vergleich zu den entsprechenden 17α-Ethinylsteroiden gefunden (EP-A-17 094).

Es wurde nun gefunden, daß die neuen 11-Methylen-$\Delta^{15}$-Steroide der allgemeinen Formel I stärker oder mindestens ebenso stark gestagen wirksam sind wie die aus der DOS 2 361 120 bekannten 11-Methylensteroide, daß sie aber im Vergleich zu den bekannten 11-Methylensteroiden eine überraschend geringe androgene Nebenwirkung haben. Bei einer Dosierung von 1 mg Substanz durch subkutane Applikation an kastrierten Rattenböcken wurden die folgenden Ergebnisse erhalten :

| Substanz | Samenblase | Prostata | Levator ani |
|---|---|---|---|
| 1) 17α-Ethinyl-17β-hydroxy-18-methyl-11-methylen-4,15-östradien-3-on | 12 | 25 | 29 |
| 2) 17α-Chlorethinyl-17β-hydroxy-18-methyl-11-methylen-4,15-östradien-3-on | 29 | 28 | 22 |
| 3) 17α-Ethinyl-17β-hydroxy-18-methyl-11-methylen-4-östren-3-on | 43 | 72 | 64 |
| (DOS 2 361 120) | | | |

Aus der Tabelle geht hervor, daß die androgene Nebenwirkung bei der Vergleichssubstanz (3) viel stärker ausgeprägt ist als bei (1) und (2).

Die Verbindungen der allgemeinen Formel I können zum Beispiel in Antikonzeptionspräparaten Verwendung finden, wobei sie als Gestagenkomponente in Kombination mit einer östrogen wirksamen Hormonkomponente, wie zum Beispiel Ethinylöstradiol, oder als alleinige Wirkkomponente eingesetzt werden. Die Verbindungen können aber auch in Präparaten zur Behandlung gynäkologischer Störungen eingesetzt werden.

Zum Gebrauch werden die neuen Verbindungen mit den in der galenischen Pharmazie üblichen Zusätzen, Trägersubstanzen und Geschmackskorrigentien nach an sich bekannten Methoden zu den üblichen Arzneimittelformen verarbeitet. Für die orale Applikation kommen insbesondere Tabletten, Dragees, Kapseln, Pillen, Suspensionen oder Lösungen infrage. Für die parenterale Applikation kommen insbesondere ölige Lösungen, wie zum Beispiel Sesamöl- oder Rizinusöllösungen infrage, die gegebenenfalls zusätzlich noch ein Verdünnungsmittel, wie zum Beispiel Benzylbenzoat oder Benzylalkohol enthalten können. Die Konzentration des Wirkstoffes ist abhängig von der Applikationsform. So enthalten beispielsweise Tabletten zur oralen Applikation vorzugsweise 0,01-0,5 mg Wirkstoff und Lösungen zur parenteralen Applikation vorzugsweise 1-100 mg Wirkstoff pro 1 ml Lösung.

Die Dosierung der erfindungsgemäßen Arzneimittel kann sich mit der Form und dem Zweck der Verabfolgung ändern. Beispielsweise liegt die tägliche kontrazeptive Dosis bei oraler Applikation bei 0,01-0,5 mg.

Die nach dem erfindungsgemäßen Verfahren eingesetzten Ausgangsverbindungen der allgemeinen Formel II können aus 11-Methylen-18-methyl-4-östren-3,17-dion (deutsche Offenlegungsschrift 2 361 120) wie folgt hergestellt werden :

15α-Hydroxy-18-methyl-11-methylen-4-östren-3,17-dion

Ein 2 Liter-Erlenmeyerkolben, der 500 ml einer 30 Minuten bei 120 °C im Autoklaven sterilisierten Nährlösung aus 3,0 % Glucose, 1,0 % Corn steep liquor, 0,2 % $NaNO_3$, 0,1 % $KH_2PO_4$, 0,2 % $K_2HPO_4$, 0,05 % $MgSO_4 \cdot 7\ H_2O$, 0,002 % $FeSO_4 \cdot 7\ H_2O$ und 0,05 % KCl enthält, wird mit einer Schrägröhrchenkultur des Stammes Penicillium patulum (ATCC 10120) beimpft und 2 1/2 Tage bei 30 °C auf einem Rotationsschüttler geschüttelt.

Mit dieser Anzuchtkultur wird ein 20 Liter-Vorfermenter beimpft, der mit 15 l eines 60 Minuten bei 121 °C und 1,1 atü sterilisierten Mediums der gleichen Zusammensetzung wie die Anzuchtkultur gefüllt ist. Unter Zugabe von Silicon SH als Antischaummittel wird bei 29 °C und 0,7 atü Druck unter Belüftung (15 l/Min.) und Rühren (220 U/Min.) 24 Stunden germiniert.

Danach werden 0,9 l dieser Kultur unter sterilen Bedingungen entnommen und damit ein 20 Liter-Hauptfermenter beimpft, der mit 14 l eines wie oben sterilisierten Nährmediums der gleichen Zusammensetzung wie die Vorfermenterkultur beschickt ist. Nach einer Anwachsphase von 12 Stunden unter Vorfermenterbedingungen wird eine Lösung von 4,5 g 18-Methyl-11-methylen-4-östren-3,17-dion in 100 ml Dimethylformamid hinzugegeben und weiter gerührt und belüftet. Der Verlauf der Fermentation wird durch Entnahme von Proben kontrolliert, die mittels Methylisobutylketon extrahiert und dünnschichtchromatographisch analysiert werden. Nach 36 Stunden Kontaktzeit ist die Umsetzung des Substrates beendet. Die Kulturbrühe wird durch Zentrifugation in einer Durchlaufzentrifuge vom Pilzmycel befreit, das klare Filtrat 3 mal mit je 10 l Methylisobutylketon extrahiert und die Extrakte mit dem Extrakt des gleichfalls mittels Methylisobutylketon extrahierten Mycels vereinigt. Nach Konzentrierung der Lösung im Umlaufverdampfer wird anschließend bei 50 °C Badtemperatur im Vakuum im Rotationsverdampfer zur Trockne eingeengt. Der ölig-kristalline Rückstand wird zur Aufreinigung über eine Kieselgelsäule

chromatographiert und mittels des Lösungsmittelgradienten 7 l Methylenchlorid-5 l Methylenchlorid/2 l Aceton eluiert. Nach Kristallisation aus Aceton erhält man 3,1 g 15α-Hydroxy-18-methyl-11-methylen-4-östren-3,17-dion vom Schmelzpunkt 228 °C.

15α-Acetoxy-18-methyl-11-methylen-4-östren-3,17-dion

2,2 g 15α-Hydroxy-18-methyl-11-methylen-4-östren-3,17-dion werden in 10 ml Pyridin mit 2,5 ml Acetanhydrid unter Argon bei Raumtemperatur innerhalb von 2 Stunden umgesetzt. Die Lösung wird in Eis/Wasser gegeben. Das ausgefallene Rohprodukt wird abgesaugt, in Essigester gelöst und getrocknet. Nach Chromatographieren des Rohproduktes an Kieselgel mit 0-20 % Aceton/Hexan werden 1,7 g 15α-Acetoxy-18-methyl-11-methylen-4-östren-3,17-dion vom Schmelzpunkt 176,3 °C erhalten.

15α-Acetoxy-3,3-(2′,2′-dimethyl-trimethylendioxy)-18-methyl-11-methylen-5-östren-17-on

5,0 g 15α-Acetoxy-18-methyl-11-methylen-4-östren-3,17-dion in 50 ml Methylenchlorid und 5 ml o-Ameisensäuretriethylester werden bei Raumtemperatur mit 10 g 2,2-Dimethyl-1,3-propandiol und 30 ml Toluolsulfonsäure versetzt. Nach 5 Stunden verdünnt man mit Methylenchlorid, wäscht mit Wasser neutral und chromatographiert das Rohprodukt an Kieselgel mit 9-12 % Aceton/Hexan. Es werden 3,9 g 15α-Acetoxy-3,3-(2′,2′-dimethyl-trimethylendioxy)-18-methyl-11-methylen-5-östren-17-on vom Schmelzpunkt 185,2 °C erhalten.

3,3-(2′,2′-Dimethyl-trimethylendioxy)-18-methyl-11-methylen-5,15-östradien-17-on

3,5 g 15α-Acetoxy-3,3-(2′,2′-dimethyl-trimethylendioxy)-18-methyl-11-methylen-5-östren-17-on werden in 30 ml Dioxan mit 2,5 ml 1,5-Diazabicyclo-(5.4.0)-undecen-(5) bei Raumtemperatur unter Argon gerührt. Nach 1 Stunde wird die Lösung in Eis/Wasser gegeben. Das ausgefallene Produkt wird abfiltriert, in Essigester gelöst, mit Wasser gewaschen und getrocknet. Nach Chromatographieren des Rohproduktes an Kieselgel mit 8-10 % Aceton/Hexan erhält man 2,1 g 3,3-(2′,2′-Dimethyl-trimethylendioxy)-18-methyl-11-methylen-5,15-östradien-17-on vom Schmelzpunkt 184,4 °C.

Mit Ethandithiol/Bortrifluorid-Etherat anstelle von 2,2-Dimethyl-1,3-propandiol/o-Ameisentriethylester/Toluolsulfonsäure erhält man 3,3-Ethylendithio-18-methyl-11-methylen-4,15-östradien-17-on.

Beispiel 1

a) 17α-Ethinyl-3,3-(2′,2′-dimethyl-trimethylendioxy)-18-methyl-11-methylen-5,15-östradien-17β-ol

Acetylen wird 30 Minuten durch eine mit Eis/Wasser gekühlte Lösung von 35 ml Butyllithium (15 %ig in Hexan) in 80 ml absolutem Tetrahydrofuran (THF) geleitet. Man gibt 1,0 g 3,3-(2′,2′-Dimethyl-trimethylendioxy)-18-methyl-11-methylen-5,15-östradien-17-on in 10 ml THF zu und rührt unter Argon. Nach 30 Minuten versetzt man das Gemisch mit gesättigter Ammoniumchloridlösung, verdünnt mit Essigester, wäscht mit Wasser und trocknet. Nach Umkristallisieren des Rohproduktes aus Aceton/Hexan werden 1,0 g 17α-Ethinyl-3,3-(2′,2′-dimethyl-trimethylendioxy)-18-methyl-11-methylen-5,15-östradien-17β-ol vom Schmelzpunkt 214,1 °C erhalten.

b) 17α-Ethinyl-17β-hydroxy-18-methyl-11-methylen-4,15-östradien-3-on

950 mg 17α-Ethinyl-3,3-(2′,2′-dimethyl-trimethylendioxy)-18-methyl-11-methylen-5,15-östradien-17β-ol werden in 20 ml Aceton bei Raumtemperatur mit 0,5 ml halbkonzentrierter Salzsäure versetzt. Nach 45 Minuten wird die Lösung mit $NaHCO_3$-Lösung neutralisiert und im Vakuum weitgehend eingeengt. Der Rückstand wird in Essigester gelöst und getrocknet. Nach Umkristallisieren aus Aceton/Hexan erhält man 370 mg 17α-Ethinyl-17β-hydroxy-18-methyl-11-methylen-4,15-östradien-3-on vom Schmelzpunkt 151,2 °C.

Beispiel 2

a) 17α-Chlorethinyl-3,3-(2′,2′-dimethyl-trimethylendioxy)-18-methyl-11-methylen-5,15-östradien-17β-ol

Zu 1,6 ml 1,2-Dichlorethylen in 10 ml absolutem Ether werden bei 0 °C und Einleiten von Argon 12 ml eine 5 %igen etherischen Methyllithiumlösung getropft. Nach 30 Minuten gibt man 700 mg 3,3-(2′,2′-Dimethyl-trimethylendioxy)-18-methyl-11-methylen-5,15-östradien-17-on in einem Gemisch aus 15 ml Ether und 5 ml THF zu und rührt bei Raumtemperatur. Nach 15 Minuten versetzt man vorsichtig mit gesättigter Ammoniumchloridlösung, verdünnt mit Ether, wäscht mit Wasser und trocknet. Es werden nach Umkristallisieren des Rohproduktes aus Aceton/Hexan 650 mg 17α-Chlorethinyl-3,3-(2′,2′-dimethyl-trimethylendioxy)-18-methyl-11-methylen-5,15-östradien-17β-ol vom Schmelzpunkt 186,6 °C erhalten.

b) 17α-Chlorethinyl-17β-hydroxy-18-methyl-11-methylen-4,15-östradien-3-on

600 mg 17α-Chlorethinyl-3,3-(2',2'-dimethyl-trimethylendioxy)-18-methyl-11-methylen-5,15-östradien-17β-ol in 20 ml Aceton werden bei Raumtemperatur unter Argon mit 0,5 ml halbkonzentrierter Salzsäure gerührt. Nach 1 1/2 Stunden wird das Gemisch mit $NaHCO_3$-Lösung neutralisiert und im Vakuum weitgehend eingeengt. Der Rückstand wird in Essigester gelöst, mit Wasser gewaschen und getrocknet. Es werden nach Umkristallisieren des Rohproduktes aus Aceton/Hexan 317 mg 17α-Chlorethinyl-17β-hydroxy-18-methyl-11-methylen-4,15-östradien-3-on vom Schmelzpunkt 149 °C erhalten.

Beispiel 3

a) 3,3-(2',2'-Dimethyl-trimethylendioxy)-18-methyl-11-methylen-17α-(1-propinyl)-5,15-östradien-17β-ol

Methylacetylen wird 30 Minuten durch eine mit Eis/Wasser gekühlte Lösung von 40 ml Butyllithium (15 %ig in Hexan) in 100 ml absolutem THF geleitet. Man gibt 2,4 g 3,3-(2',2'-Dimethyl-trimethylendioxy)-18-methyl-11-methylen-5,15-östradien-17-on in 10 ml THF zu und rührt unter Argon bei Raumtemperatur. Nach 2 Stunden tropft man in die Lösung gesättigte Ammoniumchloridlösung, verdünnt mit Essigester, wäscht mit Wasser und trocknet. Nach Chromatographieren des Rohproduktes mit 0-15 % Aceton/Hexan an Kieselgel erhält man 2,1 g 3,3-(2',2'-Dimethyl-trimethylendioxy)-18-methyl-11-methylen-17α-(1-propinyl)-5,15-östradien-17β-ol als schaumiges Produkt.

b) 17β-Hydroxy-18-methyl-11-methylen-17α-(1-propinyl)-4,15-östradien-3-on

Zu 520 mg 3,3-(2',2'-Dimethyl-trimethylendioxy)-18-methyl-11-methylen-17α-(1-propinyl)-5,15-östradien-17β-ol in 10 ml Aceton werden bei Raumtemperatur 0,2 ml halbkonzentrierte Salzsäure gegeben. Nach 30 Minuten wird die Lösung mit gesättigter $NaHCO_3$-Lösung neutralisiert und im Vakuum weitgehend eingeengt. Den Rückstand löst man in Essigester, wäscht mit Wasser und trocknet. Das Rohprodukt wird durch präparative Schichtchromatographie (Laufmittel : Ether/Chloroform 8 : 2) gereinigt. Es werden 170 mg 17β-Hydroxy-18-methyl-11-methylen-17α-(1-propinyl)-4,15-östradien-3-on erhalten.

Beispiel 4

17β-Acetoxy-17α-ethinyl-18-methyl-11-methylen-4,15-östradien-3-on

1,2 g 17α-Ethinyl-17β-hydroxy-18-methyl-11-methylen-4,15-östradien-3-on in 10 ml Pyridin werden bei Raumtemperatur unter Zugabe von 100 mg 4-Dimethylaminopyridin mit 6,5 ml Acetanhydrid umgesetzt. Das Gemisch wird nach 5 Stunden in Eis/Wasser gegeben. Das ausgefallene Produkt wird abgesaugt, in Essigester gelöst und mit Wasser gewaschen. Nach Chromatographieren des Rohproduktes an Kieselgel mit Aceton/Hexan werden 710 mg 17β-Acetoxy-17α-ethinyl-18-methyl-11-methylen-4,15-östradien-3-on als schaumiges Produkt erhalten.

Beispiel 5

17β-Butyryloxy-17α-ethinyl-18-methyl-11-methylen-4,15-östradien-3-on

500 mg 17α-Ethinyl-17β-hydroxy-18-methyl-11-methylen-4,15-östradien-3-on in 6 ml Pyridin werden bei Raumtemperatur mit 2,5 ml Buttersäureanhydrid und 120 mg 4-Dimethylaminopyridin gerührt. Nach 4 Stunden gibt man das Gemisch in Eis/Wasser, extrahiert mit Methylenchlorid und wäscht mit Wasser. Nach Chromatographieren des Rohproduktes an Kieselgel mit Aceton/Hexan werden 320 mg 17β-Butyryloxy-17α-ethinyl-18-methyl-11-methylen-4,15-östradien-3-on als Öl isoliert.

Beispiel 6

17α-Ethinyl-17β-heptanoyloxy-18-methyl-11-methylen-4,15-östradien-3-on

250 mg 17α-Ethinyl-17β-hydroxy-18-methyl-11-methylen-4,15-östradien-3-on in 4 ml Pyridin werden bei Raumtemperatur mit 2 ml Önanthsäureanhydrid und 60 mg 4-Dimethylaminopyridin 20 Stunden gerührt. Das Gemisch wird in Eis/Wasser gegeben. Es wird mit Methylenchlorid extrahiert und die Lösung mit Wasser gewaschen. Nach Chromatographieren des Rohproduktes an Kieselgel mit Aceton/Hexan erhält man 110 mg 17α-Ethinyl-17β-heptanoyloxy-18-methyl-11-methylen-4,15-östradien-3-on als Öl.

Beispiel 7

17β-Acetoy-17β-chlorethinyl-18-methyl-11-methylen-4,15-östradien-3-on

350 mg 17α-Chlorethinyl-17β-hydroxy-18-methyl-11-methylen-4,15-östradien-3-on in 5 ml Pyridin werden mit 2 ml Acetanhydrid und 20 mg 4-Dimethylaminopyridin bei Raumtemperatur umgesetzt. Nach 3 Stunden wird die Lösung in Eis/Wasser gegeben. Das ausgefallene Produkt wird abgesaugt, in Methylenchlorid gelöst und mit Wasser gewaschen. Nach Chromatographieren des Rohproduktes an Kieselgel mit Aceton/Hexan erhält man 180 mg 17β-Acetoxy-17α-chlorethinyl-18-methyl-11-methylen-4,15-östradien-3-on als schaumiges Produkt.

Beispiel 8

17β-Butyryloxy-18-methyl-11-methylen-17α-(1-propinyl)-4,15-östradien-3-on

230 mg 17β-Hydroxy-18-methyl-11-methylen-17α-(1-propinyl)-4,15-östradien-3-on in 3,5 ml Pyridin werden bei Raumtemperatur mit 2,0 ml Buttersäureanhydrid und 100 mg 4-Dimethylaminopyridin gerührt. Nach 10 Stunden wird die Lösung in Eis/Wasser gegeben. Es wird mit Methylenchlorid extrahiert, mit Wasser gewaschen und das Rohprodukt an Kieselgel mit Aceton/Hexan chromatographiert. Es werden 85 mg 17β-Butyryloxy-18-methyl-11-methylen-17α-(1-propinyl)-4,15-östradien-3-on als Öl isoliert.

**Ansprüche** (für die Vertragsstaaten : BE, CH, DE, FR, GB, IT, LI, LU, NL, SE)

1. 11-Methylen-$\Delta^{15}$-Steroide der allgemeinen Formel I

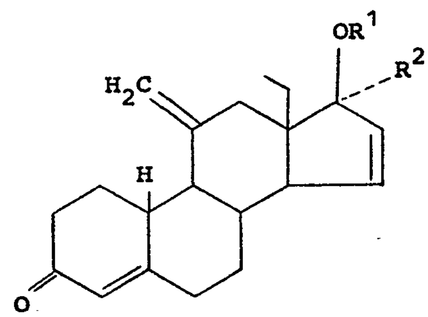

(I)

worin

$R^1$ ein Wasserstoffatom oder eine Acylgruppe und

$R^2$ eine Ethinyl-, Chlorethinyl- oder Propinylgruppe bedeuten.

2. 17α-Ethinyl-17β-hydroxy-18-methyl-11-methylen-4,15-östradien-3-on.

3. 17α-Chlorethinyl-17β-hydroxy-18-methyl-11-methylen-4,15-östradien-3-on.

4. 17β-Hydroxy-18-methyl-11-methylen-17α-(1-propinyl)-4,15-östradien-3-on.

5. 17β-Acetoxy-17α-ethinyl-18-methyl-11-methylen-4,15-östradien-3-on.

6. 17β-Butyryloxy-17α-ethinyl-18-methyl-11-methylen-4,15-östradien-3-on.

7. 17α-Ethinyl-17β-heptanoyloxy-18-methyl-11-methylen-4,15-östradien-3-on.

8. 17β-Acetoxy-17α-chlorethinyl-18-methyl-11-methylen-4,15-östradien-3-on.

9. 17β-Butyryloxy-18-methyl-11-methylen-17α-(1-propinyl)-4,15-östradien-3-on.

10. Pharmazeutische Präparate, gekennzeichnet durch einen Gehalt an Verbindungen gemäß Anspruch 1-9.

11. Verfahren zur Herstellung von 11-Methylen-$\Delta^{15}$-Steroiden der allgemeinen Formel I

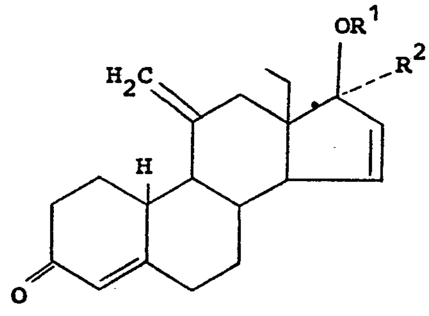

(I)

worin

$R^1$ ein Wasserstoffatom oder eine Acylgruppe und

$R^2$ eine Ethinyl-, Chlorethinyl- oder Propinylgruppe bedeuten,

dadurch gekennzeichnet, daß man in das 17-Oxo-Steroid der Formel II

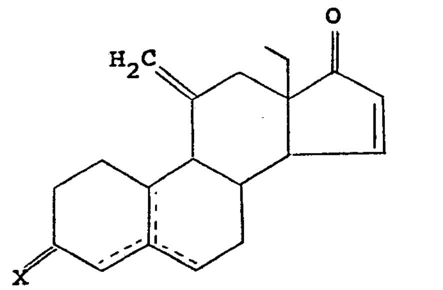

(II)

worin

X eine säurehydrolisierbare Oxoschutzgruppe darstellt und

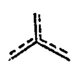 eine Doppelbindung in 4,5-, 5,6- oder 5,10-Stellung oder zwei von 3- und 5-Stellung ausgehende Doppelbindungen

bedeutet, nach an sich bekannten Methoden mit Hilfe eines den Rest $R^2$ abgebenden Mittels diesen Rest unter Bildung eines tertiären Carbinols am 17-C-Atom einführt, die 3-Oxoschutzgruppe hydrolisiert und, je nach der letzlich gewünschten Bedeutung von $R^1$, gegebenenfalls die 17-Hydroxygruppe vor oder nach der Abspaltung der Oxoschutzgruppe verestert.

**Anspruch** (für den Vertragsstaat AT)

Verfahren zur Herstellung von 11-Methylen-$\Delta^{15}$-Steroiden der allgemeinen Formel I

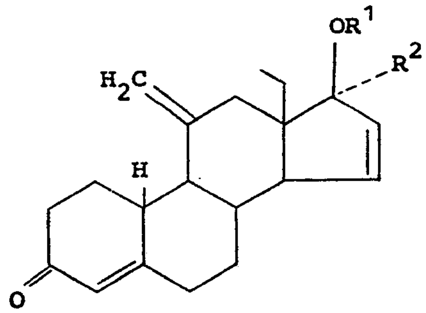

(I)

worin

$R^1$ ein Wasserstoffatom oder eine Acrylgruppe und $R^2$ eine Ethinyl-, Chlorethinyl- oder Propinylgruppe bedeuten,

dadurch gekennzeichnet, daß man in das 17-Oxo-Steroid der Formel II

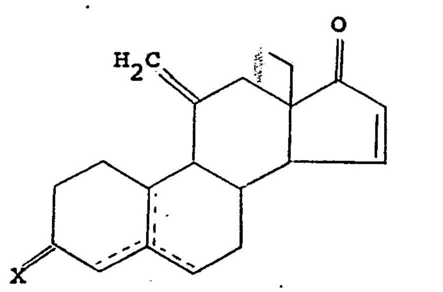

(II)

worin

X eine säurehydrolisierbare Oxoschutzgruppe darstellt und

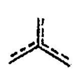 eine Doppelbindung in 4,5-, 5,6- oder 5,10-Stellung oder zwei von 3- und 5-Stellung ausgehende Doppelbindungen

bedeutet, nach an sich bekannten Methoden mit Hilfe eines den Rest $R^2$ abgebenden Mittels diesen Rest unter Bildung eines tertiären Carbinols am 17-C-Atom einfürht, die 3-Oxoschutzgruppe hydrolysiert und,

je nach der letztlich gewünschten Bedeutung von $R^1$, gegenbenenfalls die 17-Hydroxygruppe vor der nach der Abspaltung der Oxoschutzgruppe verestert.

**Claims** (for the Contracting States BE, CH, DE, FR, GB, IT, LI, LU, NL, SE)

1. 11-methylene-$\Delta^{15}$ steroids of the general formula I

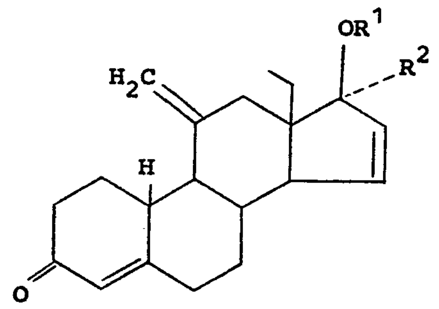

(I)

in which
$R^1$ represents a hydrogen atom or an acyl group and
$R^2$ represents an ethynyl, chloroethynyl or propynyl group.

2. 17α-ethynyl-17β-hydroxy-18-methyl-11-methylene-4,15-oestradien-3-one.
3. 17α-chloroethynyl-17β-hydroxy-18-methyl-11-methylene-4,15-oestradien-3-one.
4. 17β-hydroxy-18-methyl-11-methylene-17α-(1-propynyl)-4,15-oestradien-3-one.
5. 17β-acetoxy-17α-ethynyl-18-methyl-11-methylene-4,15-oestradien-3-one.
6. 17β-butyryloxy-17α-ethynyl-18-methyl-11-methylene-4,15-oestradien-3-one.
7. 17α-ethynyl-17β-heptanoyloxy-18-methyl-11-methtlene-4,15-oestradien-3-one.
8. 17β-acetoxy-17α-chloroethynyl-18-methyl-11-methylene-4,15-oestradien-3-one.
9. 17β-butyryloxy-18-methyl-11-methylene-17α-(1-propynyl)-4,15-oestradien-3-one.
10. Pharmaceutical preparations, characterised in that they contain compounds according to claims 1 to 9.
11. Process for the manufacture of 11-methylene-$\Delta^{15}$ steroids of the general formula I

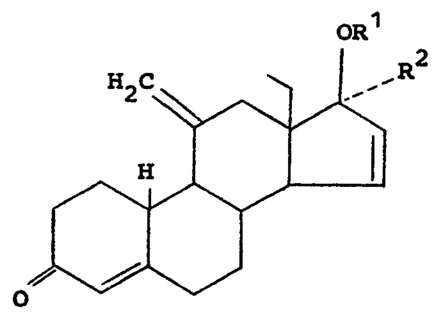

(I)

in which
$R^1$ represents a hydrogen atom or an acyl group and
$R^2$ represents an ethynyl, chloroethynyl or propynyl group,
characterised in that by methods known *per se*, with the aid of an agent that releases the radical $R^2$, that radical is introduced, with the formation of a tertiary carbinol, at the 17-carbon atom in to 17-oxo steroid of the formula II

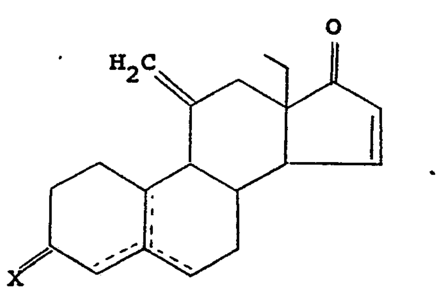

(II)

in which
X represents an oxo-protecting group that can be hydrolysed by acid and

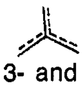 represents a double bond in the 4,5-, 5,6- or 5,10-position, or two double bonds starting in the 3- and 5-position, the 3-oxo-protecting group is hydrolysed and, depending on the desired final meaning of $R^1$, the 17-hydroxy group is optionally esterified before or after the oxo-protecting group is split off.

**Claim** (for the Contracting State AT)

Process for the manufacture of 11-methylene-$\Delta^{15}$ steroids of the general formula I

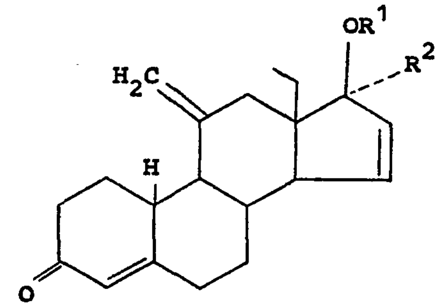

(I)

in which

$R^1$ represents a hydrogen atom or an acyl group and

$R^2$ represents an ethynyl, choroethynyl or propynyl group,

characterised in that by methods know *per se*, with the aid of an agent that releases the radical $R^2$, that radical is introduced, with the formation of a tertiary carbinol, at the 17-carbon atom into the 17-oxo steroid of the formula II

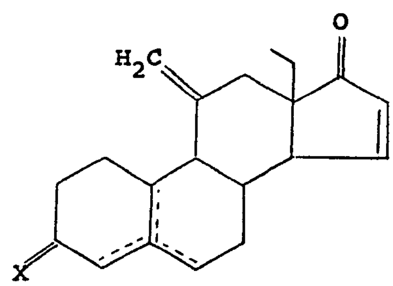

(II)

in which

X represents an oxo-protecting group that can be hydrolysed by acid and

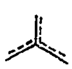 represents a double bond in the 4,5-, 5,6- or 5,10-position, or two double bonds starting from the 3- and 5-position, the 3-oxo-protecting group is hydrolysed and, depending on the desired final meaning of $R^1$, the 17-hydroxy group is optionally esterified before or after the oxo-protecting group is split off.

**Revendications** (pour les Etats contractants : BE, CH, DE, FR, GB, IT, LI, LU, NL, SE)

1. Méthylène-11 $\Delta^{15}$-stéroïdes répondant à la formule générale I

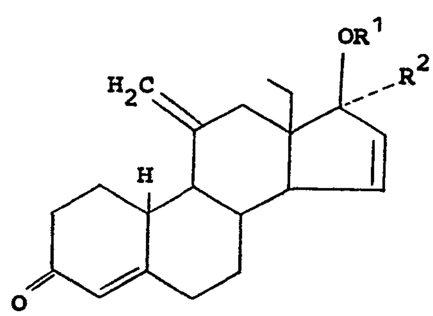

(I)

dans laquelle

$R^1$ représente un atome d'hydrogène ou un radical acyle et

$R^2$ représente un radical éthynyle, chloréthynyle ou propynyle.

2. Ethynyl-17α hydroxy-17β méthyl-18 méthylène-11 oestradiène-4,15 one-3.
3. Chloréthynyl-17α hydroxy-17β méthyl-18 méthylène-11 oestradiène-4,15 one-3.
4. Hydroxy 17β méthyl-18 méthylène-11 (propyne-1 yl)-17α oestradiène-4,15 one-3.
5. Acétoxy-17β éthynyl-17α méthyl-18 méthylène-11 oestradiène-4,15 one-3.
6. Butyryloxy-17β éthynyl-17α méthyl-18 méthylène-11 oestradiène-4,15 one-3.
7. Ethynyl-17α heptanoyloxy-17β méthyl-18 méthylène-11 oestradiène-4,15 one-3.
8. Acétoxy-17β chloréthynyl-17α méthyl-18 méthylène-11 oestradiène-4,15 one-3.
9. Butyryloxy-17β méthyl-18 méthylène-11 (propyne-1 yl)-17α oestradiène-4,15 one-3.
10. Médicaments caractérisés en ce qu'ils contiennent des composés selon l'une quelconque des revendications 1 à 9.
11. Procédé de préparation de méthylène-11 $\Delta^{15}$-stéroïdes répondant à la formule générale I

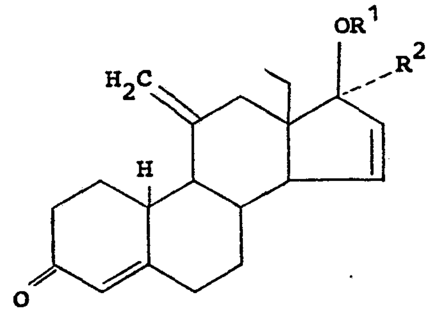

(I)

dans laquelle

$R^1$ représente un atome d'hydrogène ou un radical acyle et

$R^2$ représente un radical éthynyle, chloréthynyle ou propynyle,

procédé caractérisé en ce qu'on introduit un radical $R^2$ dans un oxo-17 stéroïde répondant à la formule II

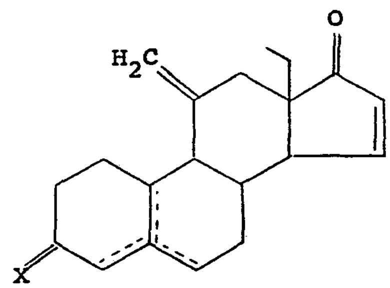

(II)

dans laquelle

X représente un radical protecteur de radical oxo qui peut être hydrolysé au moyen d'un acide et

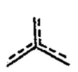 représente une double liaison en 4,5, 5,6 ou 5,10 ou deux doubles liaisons partant l'une de la position 3 l'autre de la position 5,

par des méthodes connues, au moyen d'un agent capable de céder le radical $R^2$ en question en formant un carbinol tertiaire à l'atome de carbone en 17, on hydrolyse le radical protecteur d'oxo en 3 et, suivant la signification que l'on souhaite conférer au symbole $R^1$ dans le produit final, on estérifie éventuellement le radical hydroxy en 17 avant ou après avoir éliminé le radical protecteur d'oxo.

**Revendication** (pour l'Etat contractant AT)

Procédé de préparation de méthylène-11 $\Delta^{15}$-stéroïdes répondant à la formule générale I

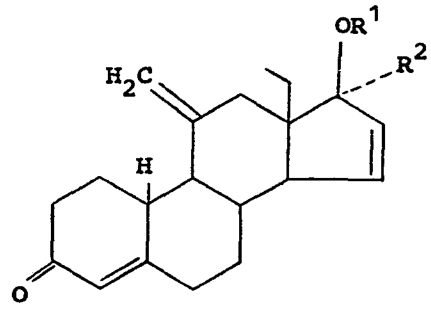

(I)

dans laquelle

$R^1$ représente un atome d'hydrogène ou un radical acyle et

$R^2$ représente un radical éthynyle, chloréthynyle ou propynyle,

procédé caractérisé en ce qu'on introduit un radical $R^2$ dans un oxo-17 stéroïde répondant à la formule II

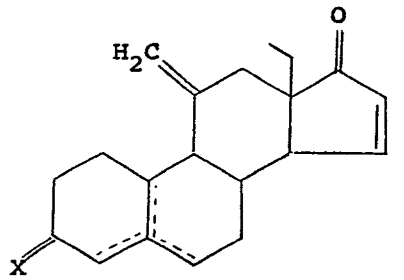

(II)

dans laquelle

X représente un radical protecteur de radical oxo qui peut être hydrolysé au moyen d'un acide et

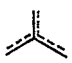 représente une double liaison en 4,5, 5,6 ou 5,10 ou deux doubles liaisons partant l'une de la position 3 l'autre de la position 5, par des méthodes connues, au moyens d'un agent capable de céder le radical $R^2$ en question en formant un carbinol tertiaire à l'atome de carbone en 17, on hydrolyse le radical protecteur d'oxo en 3 et, suivant la signification que l'on souhaite conférer au symbole $R^1$ dans le produit final, on estérifie éventuellement le radical hydroxy en 17 avant ou après avoir éliminé le radical protecteur d'oxo.